# EUROPEAN PATENT SPECIFICATION

(11) **EP 3 485 943 B1**
(45) Date of publication and mention of the grant of the patent: **14.06.2023**
(21) Application number: 18206143.2
(22) Date of filing: 14.11.2018
(51) Int. Cl.: A61Q 5/08, A61Q 5/10, A61K 8/81

(54) **PROCESS AND KIT FOR ALTERING COLOUR OF HAIR**
VERFAHREN UND KIT ZUR VERÄNDERUNG DER HAARFARBE
PROCÉDÉ ET KIT DE MODIFICATION DE LA COULEUR DES CHEVEUX

(30) Priority: 17.11.2017 EP 17202295
(43) Date of publication of application: 22.05.2019
(73) Proprietor: Kao Germany GmbH, 64297 Darmstadt (DE)
(72) Inventor: Wood, Jonathan, 64297 Darmstadt (DE); Dürr, Manfred, 64297 Darmstadt (DE); Aechtner, Anja, 64297 Darmstadt (DE); Schmelz, Sandra, 64297 Darmstadt (DE)

(56) References cited:
- WO-A1-2015/007916
- WO-A1-2016/016441
- WO-A2-2012/019688
- FR-A1- 2 848 109
- JP-A- 2013 053 119
- DATABASE GNPD [Online] MINTEL; December 2012 (2012-12), Anonymous: "2 Minute Root Touch-Up", XP002780432, retrieved from www.gnpd.com Database accession no. 1961689

## Description

The present invention relates to a process for altering colour of hair, especially human hair.

Hair dyeing and bleaching is commonly carried out by applying onto hair dyeing and/or bleaching compositions prepared by mixing several liquids and/or powder compositions and after leaving them certain period of time on the hair (processing time), the hair is rinsed off. Despite cosmetic companies offer ready to use compositions or well-designed mixed compositions which may have the required consistency to stay on the hair during the processing time, there are a lot of problems occurring when the compositions are prepared by mixing several compositions, and especially when mixing several categories of the compositions, such as bleaching compositions with dyeing compositions, or an oxidizing composition with dyeing and/or bleaching composition. One requirement for optimum bleaching and dyeing hair is that the composition is adhered well to the hair so that all ingredients may have equal staying time on hair in order to interact satisfactorily with hair. In case of insufficient consistency, this is very difficult to realize, because the composition with insufficient consistency runs away from hair so that at least one part is not enough processed, and, therewith, the hair is either inhomogeneously coloured or inhomogeneously bleached.

The problem is aggravated in streak coloring processes as the aim of the hair dressing service is to colour only the streaks and the remaining hair must stay uncolored and/or its natural color is unaltered. In case the consistency of dyeing/bleaching composition is not satisfactory, on one hand, the composition runs away so that it drips off from hair and, on the other hand, the hair fibres around the hair streak are contaminated so that a clear streak colouring may not be carried out. Often for realizing clean hair streak colouring, the hair bundle must be packed into a foil in order to keep the composition applied at its place and prevent contamination onto other fibres.

On the other hand, consumers allowing colouring their hair often, if not always, have mixture of natural and damaged hair. It is known that dye uptake of hair is very much dependent upon the physicochemical status of hair and therefore, dyeing such hair, particularly streaking, delivers even more problem when carrying out such services with compositions not having optimal consistency.

In the recent years, the consumer's demands exceed the color results achieved with the conventional dyeing compositions. In order to meet the consumers will, hair cosmetic companies started to provide various compositions which may be added to the conventional hair dyeing composition prior to application onto hair. These products vary in their compositions largely so that the consistency of the compositions changes, which is totally unexpected by the hair dressers. On the other hand, hair dressers are artists willing to achieve new hair colours on hair in order to increase consumer's satisfaction so that they wish to have the freedom to produce new compositions by mixing various colour shades and/or adding other compositions. Such kind of actions at the time of use brings about disadvantageous on the application properties of the obtained colouring mixture and/or finally, the targeted results on the hair may not be achieved.

Recently, the applicant has discovered while developing a new colour additive for obtaining so called moving colours - colour reflection changes with hair movement - addition of a colour concentrate does provide the moving colour effect, but, at the same time consistency of the new composition obtained changes in such a way that in quite large number of cases obtaining the optimum colour result may be endangered. In order to overcome this, a new composition must be developed in order to keep the consistency of obtained composition correctly adjusted so that optimum colour result may be provided.

Hair dyeing and/or bleaching with compositions having insufficient consistency results in inhomogeneous and/or insufficient dyeing and/or bleaching hair.

EP 3021828 discloses dyeing compositions comprising particular amphoteric surfactants and particular thickener. EP 2598435 provides thickening polymer comprising oxidizing and dyeing compositions. JP 2013-053119 is on thickened acidic dye, thickener, oil and penetration agent comprising composition. EP 1961451 is on thickened hair dye and bleaching compositions comprising acrylate copolymer type of thickener. None of the documents discloses and/or suggests a dyeing and/or bleaching composition comprising hydroxyalkyl acrylate copolymer.

The present invention starts from the above problems and provides a process for hair dyeing/bleaching in order to obtain satisfactory consistency which then delivers wished colour result and the streak colouring may be carried out without using foils.

The present inventors have found out that a composition comprising a copolymer of hydroxy alkyl acrylate and/or its alkaline salts provides sufficient consistency to the mixed hair dyes and/or bleaches so that optimum consistency may be achieved which secures homogeneous and intense dyeing and/or bleaching of hair. Therefore, the first object of the present invention is a process for colouring hair wherein a composition obtained from the mixture of the compositions A, B and D, wherein
a composition (Composition A) comprising one or more of the ingredients selected form one or more hair direct dyes, one or more oxidative dye precursors, one or more persalts, and one or more alkalizing agent, and having a pH in the range of 8 to 11 is mixed with an aqueous composition (Composition B) comprising one or more oxidizing agent and having a pH in the range of 2 to 6 and the mixture of Compositions A and B is added an aqueous composition (Composition D) comprising a copolymer of hydroxyalkyl acrylic acid and/or its salts,
is applied onto hair and after processing 1 to 45 min, rinsed off from hair.

The second object of the present invention is a process for colouring hair wherein a composition obtained from the mixture of compositions A, B, C and D, wherein
- a composition (Composition A) comprising one or more of the ingredients selected form one or more hair direct dyes, one or more oxidative dye precursors, one or more persalts, and one or more alkalizing agent, and having a pH in the range of 8 to 11 is mixed with an aqueous composition (Composition B) comprising one or more oxidizing agent and having a pH in the range of 2 to 6 and the mixture of Compositions A and B is added an anhydrous composition (Composition C) comprising one or more hair direct dyes in an organic solvent, and the composition thus obtained is added a composition (Composition D) comprising a copolymer of hydroxyalkyl acrylic acid and/or its salts, is applied onto hair and after processing 1 to 45 min, rinsed off from hair.

The fourth object of the present invention is a kit for hair comprising a first composition (Composition A) comprising one or more of the ingredients selected form
- One or more hair direct dye,
- One or more oxidative dye precursors,
- One or more persalts,
- One or more alkalizing agent,
and having a pH in the range of 8 to 11, a second composition (Composition B) which is an aqueous composition comprising one or more oxidizing agent and having a pH in the range of 2 to 6 , a third anhydrous composition (Composition C) comprising one or more hair direct dyes in an organic solvent, preferably one or more diol, and an aqueous composition (Composition D) comprising a copolymer of hydroxyl alkyl acrylic acid and/or its salts

The present description also discloses the use of an aqueous composition (Composition D) comprising a copolymer of hydroxyl alkyl acrylic acid and/or its salts for thickening aqueous composition obtained by mixing a composition (Composition A) comprising one or more of the ingredients selected form
- One or more hair direct dye,
- One or more oxidative dye precursors,
- One or more persalts,
- One or more alkalizing agent,
and having a pH in the range of 8 to 11 and an aqueous composition (Composition B) comprising one or more oxidizing agent and having a pH in the range of 2 to 6, which is optionally mixed with anhydrous composition (Composition C) comprising one or more hair direct dyes in an organic solvent, preferably one or more diol.

The compositions are added an aqueous composition comprising the copolymer of hydroxyalkyl acrylic acid and/or its salts. The most preferred polymer is hydroxyethyl acrylate/ acryloyl dimethyl taurate copolymer and/or its salts, preferably sodium salt. The polymer is available under the trade name Sepinow EMT 10 as a powder polymer and Sepiplus S from Seppic which is a mixture of sodium hydroxyethyl acrylate/ acryloyl dimethyl taurate, hydrogenated polyisobutane, PEG-7 trimethyl propane coconut ether, sorbitan isostearate and water.

The ready to use composition to be applied onto hair comprises the polymer at a concentration in the range of 0.1 to 10%, preferably 0.25 to 7.5%, more preferably 0.5 to 5%, most preferably 1 to 3.5%, by weight, calculated to the total of the ready to use composition.

The Composition D which is added to the mixture of compositions comprises the polymer at a concentration in the range of 20 to 70%, preferably 30 to 60%, more preferably 35 to 60%, most preferably 40 to 50% by weight calculated to the total of the Composition D. It should be noted that the Composition D is added to the Composition A which is optionally mixed with Composition C or to the mixture of the compositions A, B and optionally C at a concentration 10%by weight or less, calculated to the total of the mixture of the compositions A and optionally C or A, B and optionally C.

The composition D comprises preferably an additional silicone surfactant, preferably at a concentration in the range of 1 top 20%, more preferably 2.5 to 17.5%, most preferably 5 to 15% by weight calculated to the total of the Composition D.

The suitable silicone surfactants are ethoxylated dimethicones with 3 to 50 ethoxy units, preferably 5 to 20 ethoxy units. The non-limiting examples are PEG-3 Dimethicone, PEG-6 Dimethicone, PEG-7 Dimethicone, PEG-8 Dimethicone, PEG-9 Dimethicone, PEG-10 Dimethicone, PEG-12 Dimethicone, PEG-14 Dimethicone and PEG-17 Dimethicone. The preferred are PEG-7 Dimethicone, PEG-8 Dimethicone, PEG-9 Dimethicone, PEG-10 Dimethicone and PEG-12 Dimethicone. The more preferred are PEG-8 Dimethicone, PEG-9 Dimethicone and PEG-10 Dimethicone. The most preferred is PEG-9 Dimethicone.

The composition D comprises preferably less than 20%, more preferably less than 15% and most preferably in the range of 1 to 10% by weight water, calculated to the total of the Composition D.

The Composition A is a composition comprising one or more of the ingredients selected from:
- One or more hair direct dye,
- One or more oxidative dye precursors,
- One or more persalts, and
- One or more alkalizing agent, and
having a pH in the range of 8 to 11.

One or more hair direct dyes are selected from anionic, cationic and neutral ones. Suitable anionic direct dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27, DC Yellow 10, HC Blue 18, HC Red 18, and HC Yellow 16.

Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 and Basic Orange 31. The most preferred ones are Basic Red 51, Basic Yellow 87 Basic Orange 31, HC Blue 17 and Basic Blue 124.

Suitable neutral dyes including nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

The most preferred dyes are HC Blue 18, HC Red 18, and HC Yellow 16.

The composition comprises one or more hair direct dyes at a total concentration of 0.001% to 10%, preferably 0.005% to 9%, and more preferably 0.01% to 7.5% by weight, calculated to the total of the composition A. The composition can also comprise a mixture of several direct dyes, i.e., anionic, cationic and/or nonionic ones. In such a case the dyes may be mixed at any ratio with each other.

The composition A comprises one or more oxidative dye precursors and optionally one or more coupling substances.

Suitable oxidative dye precursors classes are p-phenylendiamines, p-aminophenols, and heterocyclic compounds such as diaminopyrazols and substituted pyrimidines, and suitable coupling substances are resorcinols, m-aminophenols, m-phenylendiamines, pyridines and substituted derivatives, and naphthols.

Non-limiting examples of the oxidative dye precursor compounds are p-phenylenediamine, p-aminophenol, 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof, and mixture thereof.

The total concentration of the dye precursors (developing substances) customarily ranges between 0.001 to 5%, preferably 0.01 to 4% and more preferably 0.05 to 3%, and most preferably 0.1 to 2% by weight, calculated to the total composition.

The aqueous composition comprising the oxidative dye precursor may further comprise coupling substances. The suitable non-limiting examples of the coupling substance if present in the composition A are 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl-resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hy-droxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof and mixture thereof.

The coupling substance are present in approximately the same molecular proportions as the developing substances, i.e. at a total concentration in the range of 0.001 to 5%, preferably 0.01 to 4% and more preferably 0.05 to 3%, and most preferably 0.1 to 2% by weight, calculated to the total composition.

In a particular embodiment, the composition A comprises one or more alkalizing agents. Suitable ones are ammonia and alkyl- or alkanolamines according to the general structure 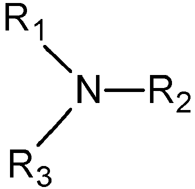 wherein R₁, R₂, and R₃ are same or different H, from C₁ to C₄, C₃ to C₄ unsaturated alkyl, C₃ to C₄ branched alkyl, C₁ to C₄ hydroxyl alkyl, C₃ to C₄ unsaturated hydroxyl alkyl, C₃ to C₄ branched hydroxyl alkyl, with the condition that at least one of R₁, R₂, or R₃ is different from H, wherein the alkalizing agents preferably selected from ammonia, monoethanolamine, and aminomethyl-propanol, and particularly suitable ones are aminomethyl-propanol and monoethanolamine.

The alkalizing agent is comprised in the composition A at a total concentration of 0.25% to 15%, preferably 0.5% to 12.5%, more preferably 0.75% to 10% and most preferably 1% to 7.5% by weight calculated to the total of the first aqueous composition.

The compositions comprising one or more direct dyes, one or more oxidative dye precursors and one or more alkalizing agent and the mixtures thereof may suitably be an aqueous composition as well as anhydrous composition. Conventionaly such kinds of compositions are aqueous which is also the preferred type of the composition A in the present invention.

The composition comprises one or more persalts. In case that the persalts are comprised, the composition is an anhydrous composition. The term anhydrous means that the composition does not comprise any added water. It should be noted that one or more ingredients of the anhydrous composition may comprise bound crystal water or residual moisture which is not covered by the term.

The anhydrous composition A comprises one or more persalts. Useful are sodium persulfate, potassium persulfate, ammonium persulfate, earth alkali peroxides such as magnesium peroxide, melamine peroxide or urea peroxide or phthalimidoperoxy hexanoic acid. The preferred persalts are sodium, potassium and ammonium persulfate. The persalt is comprised in the composition A at a total concentration in the range of 10 to 80%, preferably 15 to 70%, more preferably 20 to 60% and most preferably 25 to 60% by weight, calculated to total of composition A.

According to the invention, the anhydrous composition A can also comprise one or more ammonium salts other than persulfates at a concentration from 0.1% to 10% by weight calculated to the total of composition A. Suitable ammonium salts are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate. Compositions may also comprise mixtures of ammonium salts.

Preferred thereof are the ammonium phosphates, such as ammonium dihydrogen phopshate, diammonium hydrogen phosphate, diammonium sodium phosphate, ammonium sodium hydrogen phosphate or ammonium disodium phosphate, ammonium chloride, ammonium sulfate and diammonium hydrogen citrate.

The anhydrous composition A comprises preferably sodium metasilicate as an alkalizing agent which is preferably comprised at a concentration from 1 to 20% by weight calculated to the total of composition A.

The pH of the aqueous composition A is in the range of 8 to 11 and more preferably 8 to 10.5 and most preferably 8.5 to 10 measured at 20°C.

In addition to the above-mentioned alkalizing agents, alkali carbonates may as well be comprised in the composition A. Suitable ones are carbonate and bicarbonate alkali salts such as sodium, potassium and ammonium salts. The preferred are bicarbonate salts and especially preferred is ammonium bicarbonate.

The carbonates are comprised at a total concentration in the range of 0.25% to 10%, preferably 0.5% to 7.5%, more preferably 0.75% to 5% and most preferably 1% to 4% by weight calculated to the total of the first aqueous composition.

The composition B is an aqueous composition and comprises one or more oxidizing agent(s). The oxidizing agents suitable are hydrogen peroxide, urea peroxide, melamin peroxide or perborate salts. The most preferred is hydrogen peroxide. The composition B comprises one or more oxidizing agents at a total concentration of 1 to 20% by weight, preferably 2 to 15%, more preferably 2 to 12% and most preferably 3 to 12% by weight, calculated to total of composition B.

The pH of the aqueous composition B is in the range of 2 to 6, preferably 2.5 to 5, and more preferably 3 to 5 measured at 20°C.

The third composition, Composition C, is an anhydrous composition and comprises one or more hair direct dyes in an organic solvent, preferably the organic solvents are one or more diol(s) which is (are) liquid at 20°C, and comprised at a total concentration more than or equal to 50% by weight, preferably in the range of 55 to 90% by weight, and more preferably 60 to 90% by weight, calculated to the total of the composition C. The composition is anhydrous and comprises 2% by weight or less, preferably 1% by weight or less water, calculated to the total of the composition C.

The composition C comprises one or more diols selected from C₂ - C₆ diols, and linear or branched polymerization products of ethylene oxide and/or propylene oxide with a terminal OH group at both ends, and mixtures thereof.

Suitable C₂ - C₆ diols are ethylene glycol, propylene glycol, butylene glycol, pentylene glycol and hexylene glycol.

Suitable linear or branched polymerization products of ethylene oxide with a terminal OH group at both ends are PEG-4, PEG-6, PEG-7, PEG-8, PEG-9, PEG-10 and PEG-12.

Suitable linear or branched polymerization products of propylene oxide with a terminal OH group at both ends are PPG-3, PPG-6, PPG-7 and PPG-9.

The most preferred diol is propylene glycol also known as 1,2- propylene glycol and 1,3- propylene glycol. Especially preferred is 1,2- propylene glycol.

The compositions A, in case it is not an anhydrous composition, and B may be in the form of a solution, thickened gel or an emulsion. Emulsion form is particularly preferred.

The emulsion compositions comprise one or more fatty alcohol and one or more surfactants.

Suitable fatty alcohols are the ones with the chain length of 14 to 22 C atoms which may be saturated or unsaturated, linear or branched which may as well be substituted. Non-limiting examples are myristyl alcohol, cetyl alcohol, stearyl alcohol, behenyl alcohol, octyl dodecanol, cetostearyl alcohol, and their mixtures.

The total concentration of fatty alcohol is in the range from 0.5 to 20%, preferably 1 to 15% by weight, calculated to total of each of the composition A or B.

Compositions A, also anhydrous composition comprising the persalts, and B can further comprise surfactants selected from anionic, nonionic, amphoteric and/or cationic surfactants. The anionic, nonionic, amphoteric surfactants are used generally as emulsifier or solubilizer. The presence of surfactants may further contribute to miscibility of the compositions into another composition which may be anhydrous and/or aqueous. On the other hand, the cationic surfactants do provide the same benefits as the other surfactants but are at the same time particularly used as hair conditioners.

Anionic surfactants suitable are in principle known from the cleansing compositions. These are anionic surfactants of the sulfate, sulfonate, carboxylate and alkyl phosphate type, for example, the known C₁₀-C₁₈-alkyl sulfates, and in particular the respective ether sulfates, for example, C₁₂₋C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates. Preferred anionic surfactants are alkyl sulphate surfactants especially lauryl sulphate and its salts.

Further suitable surfactants are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, palmitic, myristic and lauric acid esters, fatty acid polyglycol esters or polycondensates of ethyleneoxide and propyleneoxide, as they are on the market, for example, under the trade name "Pluronics^{R}", as well as fatty alcohol ethoxylates, C₁₀-C₂₂-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Suitable amphoteric surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also been proven suitable.

Suitable cationic surfactants are according to the general structure 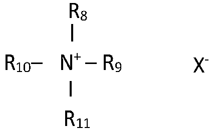
wherein R₈ is a saturated or unsaturated, branched or linear alkyl chain with 8-22 C atoms or

   R₁₂ CO NH (CH₂)ₙ
where R₁₂ is saturated or unsaturated, branched or linear alkyl chain with 7-21 C atoms and n has typical value of 1 - 4 or

   R₁₃ CO O (CH₂)ₙ
where R₁₃ is saturated or unsaturated, branched or linear alkyl chain with 7-21 C atoms and n has typical value of 1 - 4, and
R₉ is H or unsaturated or saturated, branched or linear alkyl chain with 1 - 22 C atoms or

   R₁₂ CO NH (CH₂)ₙ

   or

   R₁₃ CO O (CH₂)ₙ
where R₁₂, R₁₃ and n are same as above.

R₁₀ and R₁₁ are H or lower alkyl chain with 1 to 4 Carbon atoms, and X is typically chloride, bromide, methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The total concentration of one or more surfactants is in the range of 0.1 to 15%, preferably 0.2 to 12.5% and more preferably 0.5 - 10% by weight, calculated to the total of the compositions A or B.

The compositions A, also anhydrous composition comprising the persalts, and B may further comprise lipophilic ingredients such as vegetable oils, for example, jojoba oil or any other; liquid paraffins, especially paraffinum perliquidum and parafiinum subliquidum; silicones for example linear polysiloxanes such as dimethicones with various consistency and dimethiconols, aminated silicones with primary, secondary, tertiary or quaternary ammonium groups such as amodimethicone, polysilicone 9, and quaternium 80, cyclic silicones such as cyclomethicones, arylated silicones such as phenyl trimethicone; fatty acid esters such as octyl palmitate, isocetyl palmitate, isopropyl palmitate and octyl stearate, C₁₀- to C₃₆-fatty acid triglycerides, as well as their mixtures. Total concentration of these lipophilic compounds is in the range of 0.1 to 20% by weight, preferably from 1 to 15 percent by weight, and more preferably from 2 to 10 percent by weight, calculated to total of each of the compositions A or B.

Composition A, also anhydrous composition comprising the persalts, and B can also comprise cationic polymers as conditioning and/or thickening agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

The total concentration of cationic polymers may be in the range of 0.1 - 7.5% by weight, preferably 0.3 - 5% by weight and more preferably 0.5 - 2.5% by weight, calculated to total of each of the composition A or B.

The compositions may furthermore comprise additional thickening polymers such as polysaccharides such as alginate, pectinate, xanthan, hydroxypropyl xanthan or dehydroxanthan, non-ionic polysaccharides such as cellulose ethers (e.g., methylcellulose, hydroxyethylcellulose (HEC), methyl hydroxyethylcellulose (MHEC), ethyl hydroxyethylcellulose (EHEC), methyl ethyl hydroxyethylcellulose (MEHEC)), starch or dextrins. Synthetic acrylate type of thickeners may as well be comprised such as acrylate copolymers and alkyl acrylates homo or copolymers also know as associative thickeners.

Any of the compositions described in detail above may comprise ingredients customarily found in such compositions such as reducing agent, preservative, fragrance, chelating agent, radical scavenger, etc.

Furthermore, the present invention relates to a process for colouring hair wherein an aqueous composition obtained by mixing the composition A, B, C and D as described above, wherein compositions A and B are mixed first and optionally composition C is added to the mixture of composition A and B and finally, the composition D is added to the mixture, is applied onto hair and after processing 1 to 45 min rinsed off from hair.

The following examples are to illustrate the invention but not to limit.

### Example 1

### Thickening composition - Composition D

| | % by weight |
|---|---|
| Sepiplus S | 90 |
| PEG-9 Dimethicone | 10 |

The 0.2 part of the above composition was mixed into the mixture of oxidative dyeing composition, Composition A which did not contain Sepiplus S (replaced with water), oxidizing composition (composition B, an aqueous composition comprising 6% by weight hydrogen peroxide) and the Composition C at a weight ratio of 1:1:0.1, all by weight.

### Oxidative dyeing composition - Composition A

| | % by weight |
|---|---|
| Cetearyl alcohol | 12 |
| Sodium cetearyl sulfate | 2 |
| Cocamide MEA | 5 |
| Oleic acid | 2 |
| Tetrasodium EDTA | 1 |
| Sepiplus S | 2 |
| Sodium sulfite | 1 |
| Ammonium hydroxide | 5 |
| Ammonium chloride | 1 |
| Toluene-2,5-Diamine sulfate | 0.75 |
| Resorcinol | 0.10 |
| 4-Chlorresorcinol | 0.25 |
| m-Aminophenol | 0.05 |
| 4-Amino-2-Hydroxytoluene | 0.05 |
| Fragrance | 0.5 |
| Water | to 100 |

The above composition had a pH 9.9.

### Anhydrous dye composition - Composition C

| | % by weight |
|---|---|
| Aminomethyl propanol | 7.5 |
| HC Red 18 | 2.0 |
| HC Yellow 16 | 0.5 |
| HC Blue 18 | 0.3 |
| Fragrance | q.s. |
| 1,2-propylene glycol | to 100 |

The composition was applied onto hair and after processing 30 min at 40°C, rinsed off from hair. It was observed that the hair was coloured red-brown homogeneously and intensively. The hair colour obtained was found to be highly brilliant and showing moving reflections.

### Example 2

### Bleaching powder composition - Composition A

| | % by weight | |
|---|---|---|
| Hydroxyethylcellulose | 3 | |
| Tetrasodium EDTA | 2 | |
| Sodium carbonate | 1 | |
| Ammonium persulfate | 11 | |
| Potassium persulfate | 36 | |
| Sodium metasilicate | 10 | |
| Mineral oil | 8 | |
| Diatomaceous Earth | to 100 | |

### Oxidizing emulsion composition - Composition B

| | % by weight |
|---|---|
| Cetearyl alcohol | 2 |
| Salicylic Acid | 0.1 |
| Etidronic Acid | 0.2 |
| Phosphoric acid | 0.3 |
| Sodium Lauryl Sulfate | 1 |
| Hydrogen Peroxide | 6 |
| Water | to 100 |

The above bleaching powder composition (Composition A) was mixed with anhydrous dye composition of Example 1 (Composition C) and the above oxidizing composition (Composition B) at a weight ratio of 1:2:0.15 (by weight) and afterwards 0.25 parts of the thickening composition of Example 1 was added to the mixture and applied onto hair and after processing 30 min at ambient temperature, the hair was rinsed off and shampooed. It was observed that the hair was intensively bleached and had intensive warm red reflections.

### Example 3

### Bleaching powder composition - Composition A

| | % by weight |
|---|---|
| Hydroxyethylcellulose | 3 |
| Tetrasodium EDTA | 2 |
| Sodium carbonate | 1 |
| Ammonium persulfate | 11 |
| Potassium persulfate | 36 |
| Sodium metasilicate | 10 |
| Mineral oil | 8 |
| HC Red 18 | 1.0 |
| HC Yellow 16 | 0.25 |
| HC Blue 18 | 0.15 |
| Diatomaceous Earth | to 100 |

### Oxidizing emulsion composition - Composition B

| | % by weight |
|---|---|
| Cetearly alcohol | 2 |
| Salicylic Acid | 0.1 |
| Etidronic Acid | 0.2 |
| Phosphoric acid | 0.3 |
| Sodium Lauryl Sulfate | 1 |
| Hydrogen Peroxide | 6 |
| Water | to 100 |

The bleaching powder composition and oxidizing emulsion composition were mixed at a weight ratio of 1:2 which was then added 0.2 parts of the thickening composition of Example 1 and applied onto hair and after processing 30 min at ambient temperature, rinsed off from hair. It was observed that the hair was homogeneously bleached and had intensive warm red reflections.

## Claims

1. A process for colouring hair wherein a composition obtained from the mixture of the compositions A, B and D, wherein
- a composition (Composition A) comprising one or more of the ingredients selected from one or more hair direct dyes, one or more oxidative dye precursors, one or more persalts, and one or more alkalizing agent, and having a pH in the range of 8 to 11 is mixed with an aqueous composition (Composition B) comprising one or more oxidizing agent and having a pH in the range of 2 to 6 and the mixture of Compositions A and B is added an aqueous composition (Composition D) comprising a copolymer of hydroxyalkyl acrylic acid and/or its salts,
- is applied onto hair and after processing 1 to 45 min, rinsed off from hair.

2. A process for colouring hair wherein a composition obtained from the mixture of compositions A, B, C and D, wherein
- a composition (Composition A) comprising one or more of the ingredients selected from one or more hair direct dyes, one or more oxidative dye precursors, one or more persalts, and one or more alkalizing agent, and having a pH in the range of 8 to 11 is mixed with an aqueous composition (Composition B) comprising one or more oxidizing agent and having a pH in the range of 2 to 6 and the mixture of Compositions A and B is added an anhydrous composition (Composition C) comprising one or more hair direct dyes in an organic solvent, and the composition thus obtained is added a composition (Composition D) comprising a copolymer of hydroxyalkyl acrylic acid and/or its salts, is applied onto hair and after processing 1 to 45 min, rinsed off from hair.

3. The process according to claims 1 and/or 2 wherein the composition D comprises additionally a silicone surfactant preferably at a concentration in the range of 1 to 20%, more preferably 2.5 to 17.5%, most preferably 5 to 15% by weight calculated to the total of the Composition D.

4. The process according to claim 3 wherein the silicone surfactant is selected from ethoxylated dimethicones with 3 to 50 ethoxy units, preferably 5 to 20 ethoxy units and preferably it is selected from PEG-3 Dimethicone, PEG-6 Dimethicone, PEG-7 Dimethicone, PEG-8 Dimethicone, PEG-9 Dimethicone, PEG-10 Dimethicone, PEG-12 Dimethicone, PEG-14 Dimethicone and PEG-17 Dimethicone.

5. The process according to claims 1 to 4 **characterized in that** the composition A is an anhydrous composition.

6. The process according to claims 1 to 4 **characterized in that** the composition A is an aqueous composition.

7. The process according to claims 1 to 6 **characterized in that** the copolymer of hydroxyalkyl acrylic acid is hydroxyethyl acrylate/ acryloyl dimethyl taurate copolymer and/or its salts, preferably sodium salt.

8. The process according to claims 1 to 6 **characterized in that** the copolymer of hydroxyalkyl acrylic acid is a mixture of sodium hydroxyethyl acrylate/ acryloyl dimethyl taurate, hydrogenated polyisobutane, PEG-7 trimethyl propane coconut ether, sorbitan isostearate and water.

9. The process according to claims 1 to 8 **characterized in that** the composition D comprises the copolymer of hydroxyalkyl acrylic acid and/or its salts at a concentration in the range of 30 to 60% by weight calculated to the total of the Composition D.

10. The process according to claims 1 to 8 **characterized in that** the Composition C, is an anhydrous composition and comprises one or more hair direct dyes in an organic solvent, preferably the organic solvent is one or more diol(s) which is (are) liquid at 20°C, and comprised at a total concentration more than or equal to 50% by weight, preferably in the range of 55 to 90% by weight, and more preferably 60 to 90% by weight, calculated to the total of the composition C.

11. The process according to claim 10 **characterized in that** the diol in Composition C is selected from C₂ - C₆ diols, preferably it is selected from ethylene glycol, propylene glycol, butylene glycol, pentylene glycol and hexylene glycol and more preferably it is 1,2- propylene glycol.

12. A kit for hair comprising a first composition (Composition A), an aqueous second composition (Composition B), an anhydrous third composition, Composition C, and an aqueous composition, Composition D, wherein the compositions A, B, C and D are according to the claims 1 to 11.

## Patentansprüche

1. Verfahren zum Färben von Haaren, wobei eine Zusammensetzung, die aus der Mischung der Zusammensetzungen A, B und D erhalten wird, wobei
- eine Zusammensetzung (Zusammensetzung A), die einen oder mehrere Bestandteile, ausgewählt aus einem oder mehreren Haardirektfarbstoffen, einem oder mehreren oxidativen Farbstoffvorläufern, einem oder mehreren Persalzen und einem oder mehreren Alkalisierungsmitteln, enthält und einen pH-Wert im Bereich von 8 bis 11 aufweist, mit einer wässrigen Zusammensetzung (Zusammensetzung B), die ein oder mehrere Oxidationsmittel enthält und einen pH-Wert im Bereich von 2 bis 6 aufweist, gemischt wird und der Mischung der Zusammensetzungen A und B eine wässrige Zusammensetzung (Zusammensetzung D), die ein Copolymer von Hydroxyalkylacrylsäure und/oder deren Salzen enthält, zugesetzt wird
- auf das Haar aufgetragen und nach einer Verarbeitungszeit von 1 bis 45 Minuten vom Haar abgespült wird.

2. Verfahren zum Färben von Haaren, bei dem man eine Zusammensetzung erhält, die aus der Mischung der Zusammensetzungen A, B, C und D besteht, wobei
- eine Zusammensetzung (Zusammensetzung A), die einen oder mehrere Bestandteile, ausgewählt aus einem oder mehreren Haardirektfarbstoffen, einem oder mehreren oxidativen Farbstoffvorläufern, einem oder mehreren Persalzen und einem oder mehreren Alkalisierungsmitteln, umfasst und einen pH-Wert im Bereich von 8 bis 11 aufweist, mit einer wässrigen Zusammensetzung (Zusammensetzung B), die ein oder mehrere Oxidationsmittel umfasst und einen pH-Wert im Bereich von 2 bis 6 aufweist, gemischt wird und der Mischung der Zusammensetzungen A und B eine wasserfreie Zusammensetzung (Zusammensetzung C), die einen oder mehrere Haardirektfarbstoffe in einem organischen Lösungsmittel umfasst, zugesetzt wird und der so erhaltenen Zusammensetzung eine Zusammensetzung (Zusammensetzung D) zugesetzt wird, die ein Copolymer von Hydroxyalkylacrylsäure und/oder deren Salzen enthält, auf das Haar aufgetragen wird und nach einer Einwirkzeit von 1 bis 45 Minuten vom Haar abgespült wird.

3. Verfahren nach Anspruch 1 und/oder 2, wobei die Zusammensetzung D zusätzlich ein Silikon-Tensid enthält, vorzugsweise in einer Konzentration im Bereich von 1 bis 20 %, mehr bevorzugt 2,5 bis 17,5 %, am meisten bevorzugt 5 bis 15 Gew.-%, bezogen auf die gesamte Zusammensetzung D.

4. Verfahren nach Anspruch 3, wobei das Silikontensid ausgewählt ist aus ethoxylierten Dimethiconen mit 3 bis 50 Ethoxyeinheiten, vorzugsweise 5 bis 20 Ethoxyeinheiten, und vorzugsweise ausgewählt ist aus PEG-3 Dimethicon, PEG-6 Dimethicon, PEG-7 Dimethicon, PEG-8 Dimethicon, PEG-9 Dimethicon, PEG-10 Dimethicon, PEG-12 Dimethicon, PEG-14 Dimethicon und PEG-17 Dimethicon.

5. Verfahren nach einem der Ansprüche 1 bis 4, **dadurch gekennzeichnet, dass** die Zusammensetzung A eine wasserfreie Zusammensetzung ist.

6. Verfahren nach einem der Ansprüche 1 bis 4, **dadurch gekennzeichnet, dass** die Zusammensetzung A eine wässrige Zusammensetzung ist.

7. Verfahren nach einem der Ansprüche 1 bis 6, **dadurch gekennzeichnet, dass** das Hydroxyalkylacrylsäure-Copolymer ein Hydroxyethylacrylat/Acryloyl-Dimethyltaurat-Copolymer und/oder dessen Salze, vorzugsweise Natriumsalze, ist.

8. Verfahren nach einem der Ansprüche 1 bis 6, **dadurch gekennzeichnet, dass** das Hydroxyalkylacrylsäure-Copolymer ein Gemisch aus Natriumhydroxyethylacrylat/Acryloyldimethyltaurat, hydriertem Polyisobutan, PEG-7-Trimethylpropankokosnussether, Sorbitanisostearat und Wasser ist.

9. Verfahren nach einem der Ansprüche 1 bis 8, **dadurch gekennzeichnet, dass** die Zusammensetzung D das Hydroxyalkylacrylsäure-Copolymer und/oder seine Salze in einer Konzentration im Bereich von 30 bis 60 Gew.-%, berechnet auf die Gesamtmenge der Zusammensetzung D, enthält.

10. Verfahren nach einem der Ansprüche 1 bis 8, **dadurch gekennzeichnet, dass** die Zusammensetzung C eine wasserfreie Zusammensetzung ist und einen oder mehrere Haardirektfarbstoffe in einem organischen Lösungsmittel umfasst, wobei das organische Lösungsmittel vorzugsweise ein oder mehrere Diol(e) ist (sind), das (die) bei 20°C flüssig ist (sind), und in einer Gesamtkonzentration von mehr als oder gleich 50 Gew.-%, vorzugsweise im Bereich von 55 bis 90 Gew.-% und besonders bevorzugt 60 bis 90 Gew.-%, berechnet auf die Gesamtmenge der Zusammensetzung C, enthalten ist.

11. Verfahren nach Anspruch 10, **dadurch gekennzeichnet, dass** das Diol in der Zusammensetzung C ausgewählt ist aus C2-C6-Diolen, vorzugsweise ausgewählt aus Ethylenglykol, Propylenglykol, Butylenglykol, Pentylenglykol und Hexylenglykol und besonders bevorzugt aus 1,2-Propylenglykol.

12. Kit für Haare, umfassend eine erste Zusammensetzung (Zusammensetzung A), eine wässrige zweite Zusammensetzung (Zusammensetzung B), eine wasserfreie dritte Zusammensetzung, Zusammensetzung C, und eine wässrige Zusammensetzung, Zusammensetzung D, wobei die Zusammensetzungen A, B, C und D den Ansprüchen 1 bis 11 entsprechen.

## Revendications

1. Procédé de coloration des cheveux dans lequel une composition obtenue à partir du mélange des compositions A, B et D, dans lequel
- une composition (Composition A) comprenant un ou plusieurs des ingrédients choisis parmi un ou plusieurs colorants directs pour cheveux, un ou plusieurs précurseurs de colorants oxydants, un ou plusieurs persels, et un ou plusieurs agents alcalinisants, et ayant un pH compris entre 8 et 11 est mélangée avec une composition aqueuse (Composition B) comprenant un ou plusieurs agents oxydants et ayant un pH compris entre 2 et 6 et le mélange des Compositions A et B est additionné d'une composition aqueuse (Composition D) comprenant un copolymère d'acide acrylique hydroxyalkylique et/ou ses sels,
- est appliquée sur les cheveux et, après un traitement de 1 à 45 minutes, rincée.

2. Procédé de coloration des cheveux dans lequel une composition obtenue à partir du mélange des compositions A, B, C et D, dans laquelle
- une composition (Composition A) comprenant un ou plusieurs des ingrédients choisis parmi un ou plusieurs colorants directs pour cheveux, un ou plusieurs précurseurs de colorants oxydants, un ou plusieurs persels, et un ou plusieurs agents alcalinisants, et ayant un pH compris entre 8 et 11 est mélangée avec une composition aqueuse (Composition B) comprenant un ou plusieurs agents oxydants et ayant un pH compris entre 2 et 6 et le mélange des Compositions A et B est additionné d'une composition anhydre (Composition C) comprenant un ou plusieurs colorants directs pour cheveux dans un solvant organique, et à la composition ainsi obtenue est ajoutée une composition (Composition D) comprenant un copolymère d'acide acrylique hydroxyalkylique et/ou ses sels, est appliquée sur les cheveux et après un traitement de 1 à 45 min, est rincée des cheveux.

3. Procédé selon les revendications 1 et/ou 2, dans lequel la composition D comprend en outre un tensioactif siliconé, de préférence à une concentration comprise entre 1 et 20%, de préférence entre 2,5 et 17,5%, de préférence entre 5 et 15% en poids calculé par rapport au total de la composition D.

4. Procédé selon la revendication 3 dans lequel le tensioactif siliconé est choisi parmi les diméthicones éthoxylées ayant de 3 à 50 unités éthoxy, de préférence de 5 à 20 unités éthoxy et de préférence il est choisi parmi les PEG-3 Diméthicone, PEG-6 Diméthicone, PEG-7 Diméthicone, PEG-8 Diméthicone, PEG-9 Diméthicone, PEG-10 Diméthicone, PEG-12 Diméthicone, PEG-14 Diméthicone et PEG-17 Diméthicone.

5. Le procédé selon les revendications 1 à 4 **caractérisé par le fait que** la composition A est une composition anhydre.

6. Le procédé selon les revendications 1 à 4 **caractérisé par le fait que** la composition A est une composition aqueuse.

7. Le procédé selon les revendications 1 à 6 **caractérisé par le fait que** le copolymère d'acide acrylique hydroxyalkylique est le copolymère d'acrylate d'hydroxyéthyle/de diméthyl taurate d'acryloyle et/ou ses sels, de préférence le sel de sodium.

8. Procédé selon les revendications 1 à 6 **caractérisé par le fait que** le copolymère d'acide acrylique hydroxyalkylique est un mélange d'acrylate d'hydroxyéthyle/ acryloyl diméthyl taurate de sodium, de polyisobutane hydrogéné, d'éther de coco PEG-7 triméthyl propane, d'isostéarate de sorbitane et d'eau.

9. Le procédé selon les revendications 1 à 8 **caractérisé en ce que** la composition D comprend le copolymère d'acide acrylique hydroxyalkylé et/ou ses sels à une concentration comprise entre 30 et 60% en poids calculé par rapport au total de la composition D.

10. Procédé selon les revendications 1 à 8 **caractérisé en ce que** la composition C est une composition anhydre et comprend un ou plusieurs colorants directs pour cheveux dans un solvant organique, de préférence le solvant organique est un ou plusieurs diol(s) liquide(s) à 20°C, et compris dans une concentration totale supérieure ou égale à 50% en poids, de préférence comprise entre 55 et 90% en poids, et plus préférentiellement entre 60 et 90% en poids, calculée par rapport à la totalité de la composition C.

11. Procédé selon la revendication 10 **caractérisé en ce que** le diol de la composition C est choisi parmi les diols en C2 - C6, de préférence il est choisi parmi l'éthylène glycol, le propylène glycol, le butylène glycol, le pentylène glycol et l'hexylène glycol et plus préférentiellement il s'agit du 1,2- propylène glycol.

12. Kit pour cheveux comprenant une première composition (Composition A), une deuxième composition aqueuse (Composition B), une troisième composition anhydre, Composition C, et une composition aqueuse, Composition D, dans lequel les compositions A, B, C et D sont selon les revendications 1 à 11.
